(19) **Europäisches Patentamt / European Patent Office / Office européen des brevets**

(11) **EP 4 464 315 A1**

(12) **EUROPEAN PATENT APPLICATION**
published in accordance with Art. 153(4) EPC

(43) Date of publication:
20.11.2024 Bulletin 2024/47

(21) Application number: 22919783.5

(22) Date of filing: 26.07.2022

(51) International Patent Classification (IPC):
$A61K\ 31/19$ (2006.01)     $A61K\ 31/765$ (2006.01)
$A61P\ 19/00$ (2006.01)     $A61P\ 21/00$ (2006.01)
$A61P\ 25/00$ (2006.01)

(52) Cooperative Patent Classification (CPC):
A61K 31/19; A61K 31/765; A61P 19/00;
A61P 19/02; A61P 19/04; A61P 19/08; A61P 21/00;
A61P 25/00; Y02A 50/30

(86) International application number:
PCT/CN2022/108025

(87) International publication number:
WO 2023/134146 (20.07.2023 Gazette 2023/29)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 11.01.2022 CN 202210028046

(71) Applicant: Changchun Sinobiomaterials Co., Ltd.
Changchun, Jilin 130103 (CN)

(72) Inventors:
• WANG, Jinyue
 Changchun, Jilin 130103 (CN)
• ZHENG, Qian
 Changchun, Jilin 130103 (CN)

(74) Representative: Colombo, Stefano Paolo et al
Marchi & Partners S.r.l.
Via Vittor Pisani, 13
20124 Milano (IT)

(54) **USE OF LACTIC ACID IN PRODUCT FOR REGULATING AND PROMOTING TISSUE GROWTH**

(57) The present invention relates to regulation and promotion of body tissue growth, regeneration and healing by lactic acid or polylactic acid, and provides a novel method for preventing and treating tissue injury, which can be used for tissue injury repair, and has a broad application prospect in the aspects of tissue or organ defect treatment and repair and the like.

**EP 4 464 315 A1**

## Description

The present application claims the priority of a prior application filed with the China National Intellectual Property Administration on January 11, 2022 under the patent application number 202210028046.9 entitled "Use of Lactic Acid in Product for Regulating and Promoting Tissue Growth. "

## FIELD OF THE INVENTION

The present invention relates to the field of medical technology, in particular to the use of lactic acid in products for regulating and/or promoting tissue growth, regeneration and healing.

## BACKGROUND OF THE INVENTION

Poly-L-lactic acid (PLLA) is a biodegradable polymer material that has developed rapidly since 1990s. It is made from lactic acid as a monomer through catalytic polymerization. PLLA is widely used in the medical field. Unlike simple fillers (such as hyaluronic acid, calcium hydroxyapatite and the like), PLLA is a synthetic dermal filler that can stimulate the growth of subcutaneous collagen. Traditionally, PLLA has been thought to induce a foreign body giant cell reaction, which in turn leads to the gradual production of collagen. In our research, we found that during the degradation of PLLA, the molecular structure of PLLA is gradually destroyed and slowly hydrolyzed into lactic acid, and lactic acid can induce human fibroblasts to increase the production of collagen, leading to an increase in collagen fibers in the dermis and producing a filling and repair effect. As time increases, the dermis thickens. The PLLA in the filled area will eventually degrade into carbon dioxide and water and be replaced by new collagen, achieving a long-term cosmetic effect.

However, the application of PLLA and lactic acid and its related lactate compounds in the repair of tissues such as cartilage, connective tissue, tendon and fascia has not yet been found.

## SUMMARY OF THE INVENTION

An objective of the present invention is to provide a new use of lactic acid and its analogs. The present invention has found that lactic acid and its analogs can regulate and promote tissue regeneration, growth and healing, and can effectively prevent and treat diseases caused by tissue injury.

A first objective of the present invention is to provide the use of lactic acid and its analogs in the preparation of products for regulating and/or promoting tissue growth.

A second objective of the present invention is to provide the use of lactic acid and its analogs in the preparation of products for regulating and/or promoting tissue regeneration.

A third objective of the present invention is to provide the use of lactic acid and its analogs in the preparation of products for regulating and/or promoting tissue healing.

A fourth objective of the present invention is to provide the use of lactic acid and its analogs in the preparation of products for regulating and/or promoting tissue regeneration and/or healing.

According to a preferred technical solution of the present invention, the tissue is selected from tissues rich in collagen, more preferably, the tissue is selected from one or more of muscle tissue, connective tissue, tendon, fascia, bone, cartilage, and nerve tissue; most preferably, the tissue is selected from one or more of muscle fiber tissue, tendon, cartilage, muscle tissue, connective tissue, bone, and nerve tissue.

According to the present invention, the lactic acid and its analogs are selected from one or more of L-lactic acid degradable polymers, lactic acid and its related lactates, lactic acid compounds, and complexes of the above substances with other compounds, preferably lactic acid.

According to the present invention, the lactate is a chemical derivative of lactic acid, which is a salt formed when lactic acid releases hydrogen ions and combines with positively charged substances, including but not limited to one or more of sodium lactate, potassium lactate, lithium lactate, calcium lactate, magnesium lactate, ferrous lactate, zinc lactate, aluminum lactate, chitosan lactate, halofuginone lactate, trimethoprim lactate, 1-ethyl-3-methylimidazole L-(+)-lactate, 2-hydroxyethyl-trimethylammonium L-(+)-lactate, and L-lactic acid tetrabutylammonium salt.

According to the present invention, the product is selected from one or more of drugs, kits, health products, and medical devices.

A fifth objective of the present invention is to provide the use of lactic acid and its analogs in the preparation of drugs and/or medical devices for preventing and/or treating tissue injury.

According to a preferred technical solution of the present invention, the tissue injury is selected from injury to collagen-rich tissues. More preferably, the tissue injury is selected from injury to one or more of bone, cartilage, connective tissue, tendon, fascia, and nerve tissue. Most preferably, the tissue injury is selected from injury to one or more of muscle tissue, tendon, cartilage, and nerve tissue.

According to the present invention, the lactic acid and its analogs are selected from one or more of L-lactic acid degradable polymers, lactic acid and its related lactates, lactic acid compounds, and complexes of the above substances with other compounds, preferably lactic acid.

In a preferred embodiment of the present application, lactic acid and its analogs are active ingredients, preferably, lactic acid and its related analogs are the only active ingredients.

According to the present invention, the lactic acid and its analogs can promote cells to secrete collagen and provide energy for cell activities.

In a preferred embodiment of the present invention, the lactic acid and its analogs are injections. The dose of the lactic acid and its analogs is 25mmol/L-75mmol/L.

A sixth objective of the present invention is to provide a product for regulating and/or promoting the growth, regeneration and/or healing of a tissue and/or nerve tissue, wherein the product is made of lactic acid and its analogues and an acceptable carrier, and the tissue has the meaning as described above.

According to the present invention, the lactic acid and its analogs are selected from one or more of L-lactic acid degradable polymers, lactic acid and its related lactates, lactic acid compounds, and complexes of the above substances with other compounds; and more preferably selected from lactic acid.

According to the present invention, the product is selected from one or more of drugs, kits, health products, and medical devices.

**BENEFICIAL EFFECTS**

The present invention has found through animal experiments that lactic acid and its analogs can significantly regulate and promote tissue growth, regeneration and healing, especially for collagen-rich tissues, and thus can be used to prepare products for preventing and/or treating tissue injury, and have broad medicinal value.

**BRIEF DESCRIPTION OF THE DRAWINGS**

FIG. 1 shows the effect of injection of 25mmol/L lactic acid solution on the muscle tissue regeneration capacity of animals. The right side of the experimental mice is the experimental side (injected with lactic acid solutions of different concentrations), and the left side is the control side (injected with 0.9% sodium chloride injection).

FIG. 2 shows the effect of injection of 50mmol/L lactic acid solution on the regeneration capacity of animal muscle tissue.

FIG. 3 shows the effect of injection of 25mmol/L lactic acid solution on the growth of tendon in animals.

FIG. 4 shows the effect of injection of 50mmol/L lactic acid solution on the growth of tendon in animals.

FIG. 5 shows the effect of injection of 75mmol/L lactic acid solution on the growth of tendon in animals.

FIG. 6 shows the effect of injection of 50mmol/L lactic acid solution on the growth of ear cartilage in animals.

FIG. 7 shows the experimental results of the sciatic nerve function index.

**DETAILED DESCRIPTION OF THE INVENTION**

The technical solutions of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope of protection that the present invention intends to protect.

1. Experimental design:

Experimental animals: Male experimental mice weighing 23±4 g were selected and divided into several groups, with 10 mice in each group.

Experimental site: Different tissue sites of mice were selected for the test, specifically muscle - rectus femoris, tendon - Achilles tendon, cartilage - ear cartilage tissue.

Experimental group: ① 25mmol/L aqueous lactic acid solution

② 50mmol/L aqueous lactic acid solution

③ 75mmol/L aqueous lactic acid solution and

④ 0.9% sodium chloride injection

Experimental procedure: The right side of the experimental mouse was the experimental side (injected with lactic acid solutions at different concentrations), and the left side was the control side (injected with 0.9% sodium chloride injection).

The experiment started with a single injection of 0.1 ml of the corresponding experimental solution at the experimental side every day from 1 to 7 days. Three mice in each group were killed at 3, 7, and 14 days after surgery, respectively. The experimental and control sides of each mouse were selected for fixation and pathological histological examination.

2. Detection method:

1) The tissue to be observed was cut and fixed, and then stained with Sirius red to observe the effects of the injection solution on the tissues at various sites.

2) The experimental side and the control side were randomly selected for sectioning, the diameter of each tissue was measured under a 100x optical microscope, and the average value of the experimental side and the control side was calculated using the following formula:

Average value = (3 measurements of animal No.①+ 3 measurements of animal No.②+3 measurements of animal No. ③) / 9

The diameters on both sides were compared, and the effects of the lactic acid solution on each tissue site were analyzed using the SSPS 21 calculation results.

Example 1 Effect of lactic acid on the regeneration capacity of animal muscle tissue

1. Experimental results:

(1) Effect of injection of 25 mmol/L lactic acid solution on animal muscle tissue

Table 1 Comparison of measured values between the group injected with 25mmol/L lactate and the control group

| Timeline | Group | Mean value ($\mu$m) | Standard deviation |
|---|---|---|---|
| 3 days | Experimental group | 64.130000 | 28.1741520 |
| | Control group | 48.577500 | 17.3497697 |
| 7 days | Experimental group | 79.656250 | 15.7917736 |
| | Control group | 74.009375 | 22.8216881 |
| 14 days | Experimental group | **123.815444*** | 23.8412513 |
| | Control group | 91.573000 | 29.2364346 |
| 21 days | Experimental group | 112.073333 | 25.5295526 |
| | Control group | 99.595667 | 14.2202132 |

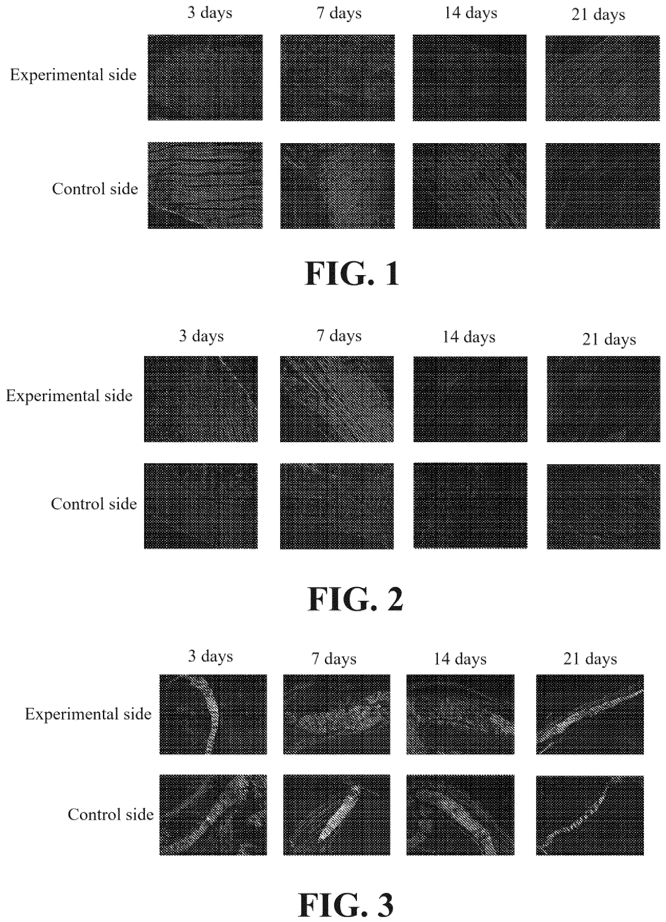

As shown in FIG. 1, in the group injected with 25mmol/L aqueous lactic acid solution, the muscle tissue on the experimental side was significantly thickened and the muscle tissue was arranged tightly at 14 days after implantation, indicating that lactic acid has the effect of promoting tissue regeneration. The values of muscle tissue measured under a 100x optical microscope are shown in Table 1: at Day 14, the experimental detection data between the experimental side and the control side were significantly different (P < 0.05).

(2) Effect of injection of 50mmol/L lactic acid solution on animal muscle tissue

Table 2 Comparison of measured values between the group injected with 50mmol/L lactate and the control group

| Timeline | Group | Mean value ($\mu m$) | Standard deviation |
|---|---|---|---|
| 3 days | Experimental group | 73.487222 | 12.3194157 |
| | Control group | 77.957889 | 27.9493057 |
| 7 days | Experimental group | **69.142125\*\*** | 10.6455341 |
| | Control group | 55.104125 | 8.1572841 |
| 14 days | Experimental group | **119.458444\*** | 26.2197044 |
| | Control group | 93.576556 | 16.2398784 |
| 21 days | Experimental group | 111.918667 | 29.0751940 |
| | Control group | 113.120500 | 20.4109819 |

As shown in FIG. 2, in the group injected with 50 mmol/L aqueous lactic acid solution, the muscle tissue on the experimental side was significantly thickened at 7 days after implantation. The values of muscle tissue measured under a 100x optical microscope are as shown in Table 2: The experimental data between the experimental side and the control side were extremely significantly different at Day 7 ($P < 0.01$), and showed significant differences at Day 14 ($P < 0.05$).

2. Conclusion

It can be seen from the above experimental results that the lactic acid solution has a significant, even extremely significant promoting effect on the regeneration of muscle tissue in animals. The muscle tissue regeneration of animals in the group injected with 25mmol/L aqueous lactic acid solution reached a significant level, and the muscle tissue regeneration of animals in the group injected with 50mmol/L aqueous lactic acid solution even reached an extremely significant level ($P<0.01$) with promoting effect.

Example 2 Effect of lactic acid on animal tendon growth

1. Experimental results:

(1) Effects of injection of 25mmol/L lactic acid solution on animal tendon tissue

Table 3 Comparison of measured values between the group injected with 25mmol/L lactate and the control group

| Timeline | Group | Mean value ($\mu m$) | Standard deviation |
|---|---|---|---|
| 3 days | Experimental group | 853.862500\* | 140.4764074 |
| | Control group | 580.665000 | 92.6874726 |
| 7 days | Experimental group | - | |
| | Control group | - | |
| 14 days | Experimental group | 877.112833 | 213.7945231 |
| | Control group | 659.691167 | 154.6656364 |
| 21 days | Experimental group | 438.280500 | 51.0615770 |
| | Control group | 427.633500 | 17.6543350 |

As shown in FIG. 3, in the 25mmol/L aqueous lactic acid solution group, the Achilles tendon on the experimental side was significantly thickened at 3 days after injection, and the diameter of the Achilles tendon was increased as detected. When detected at Day 7, the Achilles tendon was found to have multiple ruptures, so no measurement could be performed. The tendon values measured under a 100x optical microscope are shown in Table 3: The Achilles tendon of the experimental group grew rapidly at the beginning, and compared with the control group, reached a significant difference (P < 0.05).

(2) Effect of injection of 50mmol/L lactic acid solution on animal tendon tissue

Table 4 Comparison of measured values between the group injected with 50mmol/L lactate and the control group

| Timeline | Group | Mean value ($\mu m$) | Standard deviation |
|---|---|---|---|
| 3 days | Experimental group | 484.568000 | 184.0463325 |
| | Control group | 476.354500 | 60.5637965 |
| 7 days | Experimental group | 547.753000 | 95.7742615 |
| | Control group | 462.172250 | 94.4329544 |
| 14 days | Experimental group | **769.450833*** | 211.8286864 |
| | Control group | 525.717167 | 116.6757248 |
| 21 days | Experimental group | 543.914750 | 280.7437457 |
| | Control group | 523.303000 | 33.6015298 |

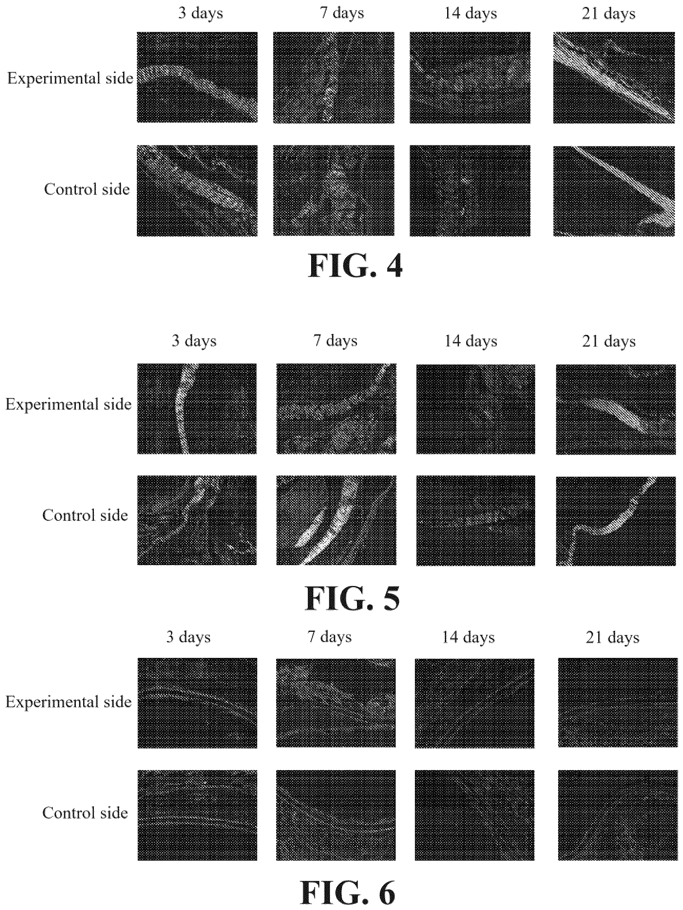

As shown in FIG. 4, in the 50mmol/L aqueous lactic acid solution group, the Achilles tendon on the experimental side was significantly thickened at Day 14, and the diameter of the Achilles tendon was increased as detected. The tendon values measured under a 100x optical microscope are shown in Table 4: A significant difference was reached between the experimental group and the control group at Day 14 (P < 0.05).

(3) Effect of injection of 75mmol/L lactic acid solution on animal tendon tissue

Table 5 Comparison of measured values between the group injected with 75mmol/L lactate and the control group

| Timeline | Group | Mean value ($\mu m$) | Standard deviation |
|---|---|---|---|
| 3 days | Experimental group | **970.857000*** | 216.6631746 |
| | Control group | 617.521000 | 57.9052828 |
| 7 days | Experimental group | **832.201500*** | 126.2168148 |
| | Control group | 514.630500 | 102.3021882 |
| 14 days | Experimental group | 578.193167 | 137.5548137 |
| | Control group | 509.734500 | 66.2128300 |
| 21 days | Experimental group | **970.090000*** | 47.5478919 |
| | Control group | 681.745500 | 207.6525129 |

As shown in FIG. 5, in the 75 mmol/L aqueous lactic acid solution group, the experimental side was significantly thickened and the diameter of the Achilles tendon increased at Day 3, 7, and 21. The tendon values measured under a 100x optical microscope are shown in Table 5: A significant difference was reached between the experimental group and the control group at Day 3, 7, and 21 (P < 0.05).

2. Conclusion:

From the above results, it can be seen that the lactic acid solution has a significant promoting effect on the growth of tendon, and as the solubility of the lactic acid solution increases, the duration of tendon growth increases, and the effect is also significant.

**Example 3 Effect of lactic acid on animal ear cartilage tissue**

1. Experimental results:

Effects of injection of 50mmol/L lactic acid solution on animal ear cartilage tissue

Table 6 Comparison of measured values between the group injected with 50mmol/L lactate and the control group

| Timeline | Group | Mean value ($\mu m$) | Standard deviation |
|---|---|---|---|
| 3 days | Experimental group | 158.793833 | 41.7279756 |
| | Control group | 144.868000 | 44.8437583 |
| 7 days | Experimental group | 237.264500 | 27.9173937 |
| | Control group | 214.423333 | 45.9045932 |
| 14 days | Experimental group | 170.348500 | 20.2403961 |
| | Control group | 214.531000 | 45.9785019 |
| 21 days | Experimental group | **253.531250*** | 16.5217714 |
| | Control group | 206.288750 | 21.8391701 |

As shown in FIG. 6, in the group injected with 50mmol/L aqueous lactic acid solution, the cross-section of the ear cartilage on the experimental side was significantly thickened at Day 21 after implantation. The values of ear cartilage tissue measured under a 100x optical microscope are shown in Table 6: at Day 21, the experimental detection data between the experimental side and the control side were significantly different (P < 0.05).

2. Conclusion:

From the above results, it can be seen that the lactic acid solution also significantly promotes the growth of animal ear cartilage tissue.

**Example 4 Effect of lactic acid on animal nerve tissue**

1. Experimental design:

Experimental animals: Male experimental mice weighing 30-35g were selected and divided into 3 groups, with 26 mice in each group.
Experimental site: The sciatic nerve of mice was selected.
Experimental groups: ① model group (injury caused by sciatic nerve clamp injury); ② treatment group (after injuried by sciatic nerve clamp, 0.1 ml of 50 mmol/L aqueous lactic acid solution was given by intraperitoneal injection each time); and ③ sham group (only the sciatic nerve was freed).
Experimental procedure: The treatment group was given medication for 14 consecutive days, and the model group and sham group were given an equal amount of 0.9% sodium chloride injection.

2. Detection method:

(1) The functional recovery of the sciatic nerve of mice after injury was tracked and evaluated by using the sciatic function index (SFI) at 7 and 14 days after surgery. Specifically, the bilateral hind feet of mice were soaked in ink and left footprints in the channel. The footprints of the left injured side (E) and the right normal side (N) of the experimental mice were measured. The footprint length (PL), the distance from the first toe to the fifth toe (TS), and the distance from the second toe to the fourth toe (IT) were recorded and put into the formula for calculation. The normal value is 0, the value is negative after nerve function injury, and the value of complete loss of function is -100. The formula is as follows:

$$SFI=109.5(ETS-NTS)/NTS-38.3(EPL-NPL)/NPL+13.3(EIT-NIT)/NIT-8.8$$

(2) The degree of toe spread on the injured side was recorded at 3, 7, and 14 days after surgery. The evaluation grades are classified as scores 0-3, with no spread being score 0 and full spread being score 3.

3. Experimental results:

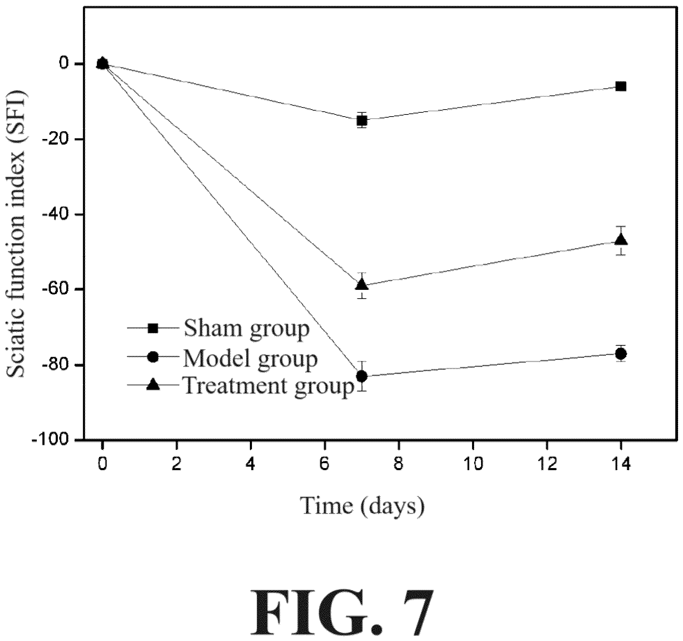

FIG. 7 demonstrates the experimental results of the sciatic nerve function index. The results showed that 7 days after surgery, the SFI scores in the model group and the treatment group were significantly lower than those in the sham group. Fourteen days after surgery, the SFI score of the treatment group was significantly increased, and the difference was statistically significant ($P<0.01$), indicating that the sciatic nerve function can be better recovered after treatment with the lactic acid solution. The toe spread experiment results in Table 7 show that the scores in the treatment group tend to be significantly higher than those in the model group, indicating that lactic acid treatment can effectively improve the toe spread function of mice.

Table 7 Scoring results of toe spread experiment in mice

|  | 3 days | 7 days | 10 days | 14 days |
|---|---|---|---|---|
| Sham group | 2.7±0.6 | 2.9±0.2 | 3.0±0.0 | 3.0±0.0 |
| Model group | 0±0.5 | 0.2±0.1 | 0.8±0.4 | 1.3±0.7 |
| Treatment group | 0±0.4 | 0.9±0.6 | 2±0.3 | 2.5±0.5 |

2. Conclusion:

From the above results, it can be seen that the lactic acid solution also significantly promotes the growth of sciatic nerve in animals.

In summary, the present invention has proved through animal experiments that the lactic acid solution has a significant promoting effect on the growth and healing of animal soft tissue, bone tissue and nerve tissue, especially muscle, tendon, cartilage tissue and sciatic nerve tissue, and can effectively prevent, treat and repair tissue injury diseases.

The embodiments of the present invention have been described above. However, the present invention is not limited to the above-mentioned embodiments. Any modifications, equivalent substitutions, improvements, and the like made within the spirit and principles of the present invention should be included in the scope of protection of the present invention.

**Claims**

1.  Use of lactic acid and its analogs in the preparation of products for regulating and/or promoting tissue growth, regeneration or healing, wherein the tissue is selected from tissues rich in collagen.

2.  The use according to claim 1, wherein the tissue is selected from at least one of muscle tissue, connective tissue, tendon, fascia, bone, cartilage, and nerve tissue;

    preferably, the tissue is selected from one or more of tendon, cartilage, muscle tissue, connective tissue, bone, and nerve tissue;
    the lactic acid and its analogs are selected from one or more of L-lactic acid degradable polymers, lactic acid and its related lactates, lactic acid compounds, and complexes of the above substances with other compounds; and
    preferably, the product is selected from one or more of medical drugs, kits, health products, and medical devices.

3.  Use of lactic acid and its analogs in the preparation of medical drugs and/or medical devices for preventing and/or treating tissue injury, wherein the tissue injury is selected from injury to collagen-rich tissues;

    preferably, the tissue injury is selected from injury to one or more of muscle tissue, connective tissue, tendon, fascia, bone, cartilage and nerve tissue; and
    most preferably, the tissue injury is selected from injury to one or more of tendon, cartilage, muscle tissue, connective tissue, bone and nerve tissue.

4. The use according to claim 3, wherein the lactic acid and its analogs are selected from one or more of L-lactic acid degradable polymers, lactic acid and its related lactates, lactic acid compounds, and complexes of the above substances with other compounds.

5. The use according to claim 3 or 4, wherein the medical drug further includes a pharmaceutically acceptable carrier.

6. The use according to any one of claims 3 to 5, wherein the lactic acid and its analogs are used as active ingredients.

7. The use according to any one of claims 3 to 6, wherein the lactic acid and its analogs are used as the only active ingredients.

8. The use according to any one of claims 3 to 7, wherein the lactic acid and its analogs are injections.

9. The use according to any one of claims 3 to 8, wherein the lactic acid and its analogs are used at a dose of 25mmol/L-75mmol/L.

10. A product for regulating and/or promoting the growth, regeneration and/or healing of a tissue and/or nerve tissue, wherein the product is made of lactic acid and its analogues and an acceptable carrier; the tissue has the meaning as described in claim 2; preferably, the lactic acid and its analogues are selected from one or more of L-lactic acid degradable polymers, lactic acid and its related lactates, lactic acid compounds, and complexes of the above substances with other compounds; and the product is selected from one or more of drugs, kits, health products, and medical devices.

**Amended claims under Art. 19.1 PCT**

1. Use of lactic acid and its analogs in the preparation of products for regulating and/or promoting tissue growth, regeneration or healing, wherein the tissue is selected from tissues rich in collagen; the dose of the lactic acid and its analogs is 0.5mmol/L-80mmol/L.

2. The use according to claim 1, wherein the tissue is selected from at least one of muscle tissue, connective tissue, tendon, fascia, bone, cartilage, and nerve tissue;

   preferably, the tissue is selected from one or more of tendon, cartilage, muscle tissue, connective tissue, bone, and nerve tissue;
   the lactic acid and its analogs are selected from one or more of L-lactic acid degradable polymers, lactic acid and its related lactates, lactic acid compounds, and complexes of the above substances with other compounds;
   preferably, the product is selected from one or more of medical drugs, kits, health products, and medical devices; and
   preferably, the dose of the lactic acid and its analogs is 25mmol/L-75mmol/L.

3. Use of lactic acid and its analogs in the preparation of medical drugs and/or medical devices for preventing and/or treating tissue injury, wherein the tissue injury is selected from injury to collagen-rich tissues;

   preferably, the tissue injury is selected from injury to one or more of muscle tissue, connective tissue, tendon, fascia, bone, cartilage and nerve tissue; and
   most preferably, the tissue injury is selected from injury to one or more of tendon, cartilage, muscle tissue, connective tissue, bone and nerve tissue.

4. The use according to claim 3, wherein the lactic acid and its analogs are selected from one or more of L-lactic acid degradable polymers, lactic acid and its related lactates, lactic acid compounds, and complexes of the above substances with other compounds.

5. The use according to claim 3 or 4, wherein the medical drug further includes a pharmaceutically acceptable carrier.

6. The use according to any one of claims 3 to 5, wherein the lactic acid and its analogs are used as active ingredients.

7. The use according to any one of claims 3 to 6, wherein the lactic acid and its analogs are used as the only active ingredients.

8. The use according to any one of claims 3 to 7, wherein the lactic acid and its analogs are injections.

9. The use according to any one of claims 3 to 8, wherein the lactic acid and its analogs are used at a dose of 25mmol/L-75mmol/L.

10. A product for regulating and/or promoting the growth, regeneration and/or healing of a tissue and/or nerve tissue, wherein the product is made of lactic acid and its analogues and an acceptable carrier; the tissue has the meaning as described in claim 2; preferably, the lactic acid and its analogues are selected from one or more of L-lactic acid degradable polymers, lactic acid and its related lactates, lactic acid compounds, and complexes of the above substances with other compounds; and the product is selected from one or more of drugs, kits, health products, and medical devices.

|  | 3 days | 7 days | 14 days | 21 days |
|---|---|---|---|---|
| Experimental side | | | | |
| Control side | | | | |

# FIG. 1

|  | 3 days | 7 days | 14 days | 21 days |
|---|---|---|---|---|
| Experimental side | | | | |
| Control side | | | | |

# FIG. 2

|  | 3 days | 7 days | 14 days | 21 days |
|---|---|---|---|---|
| Experimental side | | | | |
| Control side | | | | |

# FIG. 3

FIG. 4

FIG. 5

FIG. 6

FIG. 7

## INTERNATIONAL SEARCH REPORT

| International application No. |
|---|
| **PCT/CN2022/108025** |

**A. CLASSIFICATION OF SUBJECT MATTER**

A61K 31/19(2006.01)i; A61K 31/765(2006.01)i; A61P 19/00(2006.01)i; A61P 21/00(2006.01)i; A61P 25/00(2006.01)i

According to International Patent Classification (IPC) or to both national classification and IPC

**B. FIELDS SEARCHED**

Minimum documentation searched (classification system followed by classification symbols)

A61K 31/-; A61P 19/-; A61P 21/-; A61P 25/-

Documentation searched other than minimum documentation to the extent that such documents are included in the fields searched

Electronic data base consulted during the international search (name of data base and, where practicable, search terms used)

CNTXT; DWPI; WPABSC; ENTXTC; CNKI; 百度学术, BAIDU SCHOLAR; ISI WEB OF SCIENCE: 圣博玛生物材料, 王晋岳, 郑茜, 乳酸, 胶原, 肌肉, 结缔, 肌腱, 筋膜, 骨, 软骨, 神经, 损伤, 增长, 生长, 愈合, 再生, lactic, lactate, muscle, connective, tendon, fascia, bone, cartilage, nerve, injur+, damage+, regenerat+, heal+

**C. DOCUMENTS CONSIDERED TO BE RELEVANT**

| Category* | Citation of document, with indication, where appropriate, of the relevant passages | Relevant to claim No. |
|---|---|---|
| X | US 2018092869 A1 (NELSON DEANNA J) 05 April 2018 (2018-04-05) <br> claims 6-10 and 15-16, and description, paragraph 95 | 1-10 |
| X | CN 106102733 A (STAYBLE THERAPEUTICS AB) 09 November 2016 (2016-11-09) <br> claims 1 and 3, and description, paragraphs 16-17, 32, and 57 | 1-10 |
| X | US 2013108670 A1 (BIOMIMETIC THERAPEUTICS INC.) 02 May 2013 (2013-05-02) <br> claims 1-2 and 14, and description, paragraph 107 | 1-4, 8 |
| X | CN 1386478 A (INSTITUTE OF CHEMISTRY, CHINESE ACADEMY OF SCIENCES) 25 December 2002 (2002-12-25) <br> claims 1 and 7, and embodiment 7 | 1-6, 10 |
| X | JP 2002065247 A (TAKI CHEMICAL CO., LTD.) 05 March 2002 (2002-03-05) <br> claim 1, and description, paragraphs 21-23 | 1-4, 6-7 |
| X | JP 2000197693 A (JMS CO., LTD. et al.) 18 July 2000 (2000-07-18) <br> claims 1 and 9 | 3-4, 6-7 |
| X | EP 0649300 B1 (CENTRE NATIONAL DE LA RECHERCHE SCIENTIFIQUE (CNRS)) 05 March 1997 (1997-03-05) <br> claim 1, and description, column 1, lines 12-24, and column 4, line 36 to column 5, line 2 | 1-4, 6-7 |

☐ Further documents are listed in the continuation of Box C.    ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **13 October 2022** | **31 October 2022** |

| Name and mailing address of the ISA/CN <br><br> **China National Intellectual Property Administration (ISA/CN)** <br> **No. 6, Xitucheng Road, Jimenqiao, Haidian District, Beijing 100088, China** | Authorized officer |
|---|---|
| Facsimile No. **(86-10)62019451** | Telephone No. |

Form PCT/ISA/210 (second sheet) (January 2015)

# EP 4 464 315 A1

<div align="center">

**INTERNATIONAL SEARCH REPORT**
Information on patent family members

</div>

| International application No. |
| --- |
| **PCT/CN2022/108025** |

| Patent document cited in search report | | | Publication date (day/month/year) | Patent family member(s) | | | Publication date (day/month/year) |
| --- | --- | --- | --- | --- | --- | --- | --- |
| US | 2018092869 | A1 | 05 April 2018 | WO | 2018084959 | A2 | 11 May 2018 |
| | | | | ZA | 201902258 | B | 26 August 2020 |
| | | | | US | 2020016101 | A1 | 16 January 2020 |
| CN | 106102733 | A | 09 November 2016 | JP | 2017508813 | A | 30 March 2017 |
| | | | | LT | 3119386 | T | 10 October 2019 |
| | | | | PT | 3119386 | T | 01 October 2019 |
| | | | | US | 2018169041 | A1 | 21 June 2018 |
| | | | | ES | 2746075 | T3 | 04 March 2020 |
| | | | | IL | 247882 | D0 | 30 November 2016 |
| | | | | RU | 2016137165 | A | 26 April 2018 |
| | | | | KR | 20160125518 | A | 31 October 2016 |
| | | | | DK | 3119386 | T3 | 16 September 2019 |
| | | | | PL | 3119386 | T3 | 31 December 2019 |
| | | | | HR | P20191633 | T1 | 13 December 2019 |
| | | | | AU | 2015233396 | A1 | 29 September 2016 |
| | | | | MX | 2016012050 | A | 13 April 2017 |
| | | | | EP | 3119386 | A1 | 25 January 2017 |
| | | | | SI | 3119386 | T1 | 29 November 2019 |
| | | | | CA | 2943256 | A1 | 24 September 2015 |
| | | | | WO | 2015140320 | A1 | 24 September 2015 |
| | | | | HU | E046209 | T2 | 28 February 2020 |
| | | | | RS | 59285 | B1 | 31 October 2019 |
| | | | | US | 2018280327 | A1 | 04 October 2018 |
| US | 2013108670 | A1 | 02 May 2013 | WO | 2013062994 | A1 | 02 May 2013 |
| CN | 1386478 | A | 25 December 2002 | None | | | |
| JP | 2002065247 | A | 05 March 2002 | None | | | |
| JP | 2000197693 | A | 18 July 2000 | None | | | |
| EP | 0649300 | B1 | 05 March 1997 | JP | H08500981 | A | 06 February 1996 |
| | | | | ZA | 933946 | B | 30 December 1993 |
| | | | | FR | 2691901 | A1 | 10 December 1993 |
| | | | | ZA | 9303946 | B | 30 December 1993 |
| | | | | AT | 149342 | T | 15 March 1997 |
| | | | | AU | 4330993 | A | 30 December 1993 |
| | | | | DE | 69308561 | D1 | 10 April 1997 |
| | | | | EP | 0649300 | A1 | 26 April 1995 |
| | | | | WO | 9324097 | A1 | 09 December 1993 |

Form PCT/ISA/210 (patent family annex) (January 2015)

**15**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210028046